# EUROPEAN PATENT SPECIFICATION

(11) **EP 4 132 707 B1**
(45) Date of publication and mention of the grant of the patent: **10.04.2024**
(21) Application number: 21763051.6
(22) Date of filing: 13.08.2021
(51) Int. Cl.: B01L 3/00, B03C 1/033, B03C 1/034, B03C 1/28, C12M 1/00, G01N 33/543

(54) **METHOD AND FLOW CELL FOR SEPARATING BIOMOLECULES FROM LIQUID MEDIUM**
VERFAHREN UND DURCHFLUSSZELLE ZUR TRENNUNG VON BIOMOLEKÜLEN AUS EINEM FLÜSSIGEN MEDIUM
PROCÉDÉ ET CELLULE D'ÉCOULEMENT DE SÉPARATION DE BIOMOLÉCULES À PARTIR D'UN MILIEU LIQUIDE

(30) Priority: 18.08.2020 EP 20191584
(43) Date of publication of application: 15.02.2023
(73) Proprietor: Technische Universität München, 80333 München (DE)
(72) Inventor: SCHWAMINGER, Sebastian, 80339 München (DE); BERENSMEIER, Sonja, 81829 München (DE); FRAGA GARCÍA, Paula, 85354 Freising (DE)
(74) Representative: Vossius & Partner Patentanwälte Rechtsanwälte mbB
(86) International application number: PCT/EP2021/072622
(87) International publication number: WO 2022/038059

(56) References cited:
- EP-A1- 2 955 508
- EP-A1- 3 059 588
- WO-A1-2018/050826
- US-A1- 2004 018 611
- US-A1- 2006 186 055
- US-A1- 2012 070 858
- IHARA I ET AL: "High Gradient Magnetic Separation Combined With Electrocoagulation and Electrochemical Oxidation for the Treatment of Landfill Leachate", IEEE TRANSACTIONS ON APPLIED SUPERCONDUCTIVITY, IEEE SERVICE CENTER, LOS ALAMITOS, CA, US, vol. 14, no. 2, 1 June 2004 (2004-06-01), pages 1558-1560, XP011117647, ISSN: 1051-8223, DOI: 10.1109/TASC.2004.830706
- CLÁUDIA SG GOMES ET AL: "Magnetic hydrophobic-charge induction adsorbents for the recovery of immunoglobulins from antiserum feedstocks by high-gradient magnetic fishing : Magnetic hydrophobic-charge induction adsorbents for antibody capture from complex feeds", JOURNAL OF CHEMICAL TECHNOLOGY AND BIOTECHNOLOGY, vol. 93, no. 7, 30 March 2018 (2018-03-30) , pages 1901-1915, XP055757330, ISSN: 0268-2575, DOI: 10.1002/jctb.5599

## Description

The present invention relates to a method for separating biomolecules from a liquid medium and a flow cell with which the method can be implemented. In particular, said method and/or said flow cell involves magnetic separation and electro-desorption of biomolecules.

Bio-nanotechnology has grown into a rapidly emerging research field. This discipline combines innovative techniques and new materials with phenomena occurring in nature and proves to be useful in multiple applications. For example, interaction between biomolecules and inorganic nanoparticles can be utilized in such applications as magnetic resonance imaging, drug delivery, nanomedicine for implant coating as well as energy storage. Also, the transfer of electrons between the nanomaterials and the biomolecules can be used for biomolecule detection for e.g. antibodies or pathogens. In this regard, it is very much of interest to control and manipulate the bio-nano interface in order to improve applications in all these fields.

More specifically, it is known to extract directly from crude cell lysates a target biomolecule material by magnetic separation. The method includes modifying the target biomolecule material so as to enhance its affinity to magnetic nanoparticles and then combining the same with the magnetic nanoparticles so that the target biomolecule material may be magnetically manipulated. The target biomolecule material may thereafter be released from the magnetic nanoparticles in an elution buffer with certain pH value.

In view of the great potential, further improvements as regards magnetic separation of biomolecules have been sought to provide alternative approaches for biomolecule recognition, detection and purification.

### Non-patent literature:

Schwaminger et al., Magnetic One-Step Purification of His-Tagged Protein by Bare Iron Oxide Nanoparticles, ACS Omega 2019, 4, 3790-3799, DOI: 10.1021/acsomega.8b03348

The conventional magnetic separation of biomolecules described in the above non-patent literature requires the use of buffer/pH switch, which adds further costs. Furthermore, it could be time-consuming as the diffusion takes some time and the elution is not completed before an equilibrium is reached. The buffer-switch approach may also cause aggregation or degeneration of biomolecules.

US 2006/0186055 A1 discloses a device for separating a biological component, the device comprising magnetically responsive particles and a chip through which one or multiple channels are formed. The biological component is adsorbable to the magnetically responsive particles when contacting the same and is separable therefrom by applying an electric field. The present invention provides an innovative approach in biomolecule recognition, detection and purification. An objective of the present invention is to establish a cheaper, faster, more precise and possibly less harmful technique for biomolecule separation by using the magnetic properties of nanoparticles and the electrochemical interaction between the nanoparticles and the biomolecules. The invention is defined by the independent claims.

In a first aspect, the present invention is directed to a method for separating biomolecules from a liquid medium as defined in claim 1.

As used herein, the expression "a method for separating biomolecules from a liquid medium" covers both a concentration (or purification) process and a detection process for biomolecules. Therefore, suitable adjustment of parameters may be made, for example as regards the ratio of the biomolecules to the magnetic nanoparticles, in view of the desired result of the respective processes.

The biomolecules preferably comprise proteins, such as antibodies, antigens, vaccines, enzymes, receptors, etc. These proteins, as mentioned above, are each adapted to bind to the respective surfaces of the magnetic nanoparticles. Preferably, the adaption is realized by providing biomolecules at least in part electrically charged. More specifically, the adaption is realized by combining peptide tags to the proteins. That is, preferably, the method of the present invention further comprises a step of attaching peptide tags to the proteins so as to obtain proteins fused with the peptide tags.

As used herein, the word "comprise" may carry its open-ended meaning (i.e. as "include") and/or its close-ended meaning (i.e. as "consist of'), as long as the context allows. For example, "comprise" in the expression of "the liquid medium comprising the biomolecules" carries the open-ended meaning as the liquid medium clearly contains further components (e.g. water), while the same in the expression that "the biomolecules preferably comprise proteins" may be understood as either open-ended or close-ended.

As used herein, "peptide tags" means short peptide sequences consisting of up to 30 amino acids, which sometimes are also called binding or recognition tags. These tags facilitate a specific binding (e.g. complexation, ionic interaction, etc.) to the surface of a magnetic nanoparticle and thus allow controlled movement of proteins fused to such peptide sequences. Preferably, the peptide tags comprise charged peptide tags, and more preferably negatively charged peptide tags. Various methods for attaching such peptide tags to proteins are well known in the field and thus are not detailed herein.

Without wishing to be bound by theory, it is found that the peptide tags may form charge transfer complexes with respective surfaces of the magnetic nanoparticles and that the complex formation may be broken by a change of the electrostatic interactions. Specifically, the application of an electric potential should lead to a rearrangement of the electrochemical double layer formed at the interface between the magnetic nanoparticles and the liquid medium. This means that the surfaces of the magnetic nanoparticles could be positively or negatively charged after applying the electric potential. An electrostatic repulsion may thereby be created between the magnetic nanoparticles and the charged peptide tags (and accordingly the proteins fused with said peptide tags), which should lead to release of the proteins. For example, in case that the peptide tags comprise negatively charged peptide tags, the method of the present invention preferably comprises a step of applying a negative electric potential to the surface of the collector so as to repulse the proteins fused with the negatively charged peptide tags. The biomolecules are thereby released from the magnetic nanoparticles. Preferably, this releasing mechanism is not limited to the specific embodiment where the biomolecules comprise proteins fused with peptide tags. Rather, applying an electric potential to the surface of the collector so that the surfaces of the magnetic nanoparticles are positively or negatively charged may be effective in releasing the biomolecules as long as the binding between the biomolecules and the surfaces of the magnetic nanoparticles is realized due to biomolecules being at least in part electrically charged (e.g. as the binding is complexation, ionic interaction, etc.).

Typical examples for the peptide tags comprise glutamic acid-based tag, aspartic acid-based tags or Histidine-based tags, and in particular the (His₆)-tag, FLAG-tag, Glu₆-tag or Strep-tag. Alternatively, for the positively charged tags, arginine- or lysine-based peptides may be used.

Preferably, the liquid medium comprises human/bodily fluids, cell lysates or other cell supernatants. It may also be other liquid media in which biomolecules such as aforementioned proteins exist. These include e.g. pulp from fruit or vegetables, plant extract as well as milk/whey. Typical buffers for the cell lysates that may be used in the method of the present invention comprise phosphate-buffered saline (PBS) or Tris-buffered saline (TBS). Other buffer systems such as Good's buffers may be used as well.

Preferably, the magnetic nanoparticles comprise iron oxide nanoparticles, and more preferably superparamagnetic iron oxide nanoparticles (SPION). Various methods for synthesizing such nanoparticles are well known in the field and thus are not detailed herein.

Superparamagnetism allows the magnetic nanoparticles to be controlled in magnetic fields while not showing any magnetization (no remanence) outside of the magnetic fields. Furthermore, the iron oxide nanoparticles demonstrate a low toxicity towards most living systems. For most applications, iron oxide nanoparticles are stable and may be further modified (e.g. coated) to turn them into a system responsive to diverse stimuli such as temperature, pH, ionic strength, specific molecules and electric or magnetic fields.

As regards the iron oxide nanoparticles that may be used in the present invention, the particle size thereof is typically around 1 to 100 nm and preferably 5 to 20 nm. The aggregation size thereof is typically around 10 nm to 10 µm and preferably 80 to 1,000 nm. The saturation magnetization thereof is typically around 10 to 100 Am²/kg at around 0.1 to 1 Tesla, for example 30 Am²/kg at 0.1 Tesla and 70 to 80 Am²/kg at 1 Tesla.

Depending on whether the method of the present invention is used for detection or purification, a weight ratio of the magnetic nanoparticles to the biomolecules may preferably be 10³-10⁶ to 1 (for detection) or 1-10 to 1 (for purification).

As used herein, the term "collector" indicates a physical entity which may be brought into contact with the liquid medium comprising the biomolecules and on which the biomolecules binding to the magnetic nanoparticles may be collected with the application of a magnetic force. The method of the present invention may be implemented in various systems with different environments. Therefore, the collector herein is not limited to any particular size, shape or material as long as the aforementioned functionalities may be satisfied. For example, the method of the present invention may be implemented in a beaker while the collector may then be a plate inserted into the beaker. The method may alternatively be implemented using a flow cell comprising a chamber, as further described with respect to another aspect of the present invention below. In such a scenario, the collector may be a component separate from the chamber or a part (e.g. the inner wall) of the chamber.

As regards the magnetic field, the direction and the magnitude thereof are not particularly limited as long as the magnetic field is capable of attracting the magnetic nanoparticles bound with the biomolecules to the surface of the collector. Preferably, the magnitude of the magnetic field on the surface of the collector is at least 0.01 Tesla, more preferably at least 0.1 Tesla. For the purpose of the present invention, there is no particular upper limit for the magnitude of the magnetic field, which may reach e.g. 1 to 10 Tesla. The magnetic field may be provided by any known means such as a permanent magnet or an electric magnet.

As regards the electric potential, the magnitude thereof is not particularly limited as long as the electric potential is capable of releasing the biomolecules from the collector. The electric potential at the surface of the collector may be in a range of -10 to 10 V, preferably in a range of -1 to 1 V. Stronger potentials may lead to oxidation of the biomolecules as well as the formation of hydrogen, which might endanger the system.

Preferably, in the method of the present invention, step ii) comprises allowing the liquid medium to which the magnetic particles have been added to pass through a flow cell comprising a chamber and the collector, the collector being disposed in the chamber. As mentioned above, the expression of "the collector being disposed in the chamber" covers both a configuration where the collector is a component separate and independent from and disposed in the chamber and a configuration where the collector is a part of the chamber.

Preferably, the method of the present invention further comprises, before step iv), separating the collector from the liquid medium and bringing at least part of the collector into contact with another liquid medium. For example, when the method of the present invention is implemented in a beaker as mentioned above, the collector on which the magnetic nanoparticles have already been collected may be removed from the beaker containing the liquid medium and disposed into another container (e.g. another beaker) which contains another liquid medium (e.g. a buffer), in which the elution by step iv) may then be carried out.

Preferably, the method of the present invention further comprises a step of removing the magnetic field to release the magnetic nanoparticles from the collector. Preferably, the method of the present invention further comprises a step of collecting the magnetic nanoparticles released from the collector. This preferably occurs after the release of the biomolecules from the magnetic nanoparticles and allows the magnetic nanoparticles to be recycled.

Preferably, the method of the present invention further comprises a step of collecting the biomolecules released from the magnetic nanoparticles. This allows further processing of the biomolecules.

In a second aspect, the present invention is directed to a flow cell for separating biomolecules from a liquid medium. The flow cell comprises a chamber, the chamber comprising an inlet and an outlet which define therebetween a fluid path for the liquid medium. The chamber further comprises a volume for containing the liquid medium provided along said fluid path. The volume comprises a collecting area for biomolecules. The flow cell further comprises a magnetic source proximal to the volume of the chamber. The magnetic source is arranged and configured to generate a magnetic field extending at least between the collecting area and the remaining volume for containing the liquid medium. The flow cell further comprises a working electrode proximal to the volume of the chamber. The working electrode is arranged and configured to generate an electric field at the collecting area.

As used herein, a "flow cell" is not particularly limited in terms of its shape, dimension or material as long as the chamber, the inlet and the outlet thereof are configured to allow the method of the present invention to be implemented. For example, the flow cell may be a structure that allows the method to be implemented continuously or a structure that allows the method to be implemented batch-wise. That is, the chamber of the flow cell is configured in a manner that when the flow cell is used for separating the biomolecules from the liquid medium, the liquid medium would be at least temporarily held within the volume of the chamber. The flow cell may also be a structure for microscopic treatment or a structure for a treatment of a much larger scale.

As used herein, the "collecting area" is a space in the chamber in which the biomolecules will be collected and from which the biomolecules will be subsequently released when the flow cell is used for separating the biomolecules from the liquid medium. For example, the collecting area may be a portion of the inner wall of the chamber when the magnetic source is configured to attract the biomolecules onto the wall of the chamber. Alternatively, when a collector which is an additional component to the chamber is used in the method of the present invention as described above, the collecting area will be defined by a surface of the collector.

As used herein, the expression "proximal to the volume" indicates that the element concerned, i.e. the magnetic source or the working electrode, is disposed within a sufficiently close distance to the volume so that a magnetic or electric field sufficient for carrying out the method of the present invention may be established at the collecting area. Therefore, this expression encompasses a configuration where the element concerned is not in direct contact with, but is adjacent to, the volume and also a configuration where the element concerned is in direct contact with the volume. For example, the working electrode may be disposed in the chamber. In such a scenario, the working electrode is in direct contact with the volume of the chamber and may serve as the collector as described above.

Preferably, the volume for containing the liquid medium along said fluid path coincides with the inner volume of the chamber. Thus, it is preferred that the magnetic source is arranged and configured to generate a magnetic field extending at least between the collecting area and the remaining inner volume of the chamber so as to attract any magnetic particles within said inner volume. However, the volume for containing the liquid medium along said fluid path may also be smaller than and encompassed by the inner volume of the chamber. For example, it may be sufficient if the magnetic field generated by the magnetic source extends from a collecting area at an edge of the inner volume of the chamber through an entire cross-section of said inner volume so as to attract all magnetic particles flowing through said cross-section, i.e. passing by said collecting area. It is thus preferred that the volume for containing the liquid medium along said fluid path covers a full cross section of the inner volume of the chamber along substantially the length of the collecting area.

As regards the magnetic source, no particular limitation is required as long as it is capable of creating a magnetic field extending at least between the collecting area and the rest of the volume of the chamber. Such a magnetic field is capable of attracting magnetic nanoparticles from the rest of the volume of the chamber to the collecting area. Said magnetic field may correspond to the magnetic field described above with respect to the method of the present invention.

Preferably, the magnetic source is a permanent magnet movably coupled to the working electrode and/or to the chamber. Alternatively, the magnetic source may be an electromagnet. This allows a magnetic field thereby produced to be adjusted and/or switched on and off.

As regards the working electrode, no particular limitation is required as long as it is capable of creating an electric field extending at least between the collecting area and the rest of the volume of the chamber. Such an electric field may establish at the collecting area the electric potential described above with respect to the method of the present invention.

The flow cell of the present invention may comprise additional functional units. For example, the flow cell preferably comprises a first collecting unit in fluid communication with the outlet of the chamber for collecting the biomolecules from the liquid medium, allowing the biomolecules to be further treated. Preferably, the flow cell further comprises a second collecting unit in fluid communication with the outlet of the chamber for collecting a magnetic material from the liquid medium, allowing the magnetic nanoparticles to be recycled. Preferably, the flow cell further comprises a supply unit in fluid communication with the inlet of the chamber for supplying the liquid medium to the chamber. Pre-treatments of the biomolecules in the liquid medium may be carried out in the supply unit. These include e.g. attaching peptide tags as mentioned above to the biomolecules and/or allowing the biomolecules to bind to the magnetic nanoparticles.

Preferably, the flow cell of the present invention is a microfluidic chip, which allows a precise control and manipulation of fluid in a small scale (e.g. sub-millimeter to millimeter). Preferably, a volume of the chamber of the microfluidic chip ranges from 1 µm³ to 1,000 mm³, more preferably 1 µm³ to 100 mm³, and even more preferably 1 µm³ to 10 mm³.

Preferably, the microfluidic chip further comprises a counter electrode, wherein the working electrode and the counter electrode of the microfluidic chip are respectively disposed on opposite walls of the chamber. The working electrode is in direct contact with the volume of the chamber and serves as a collector on which the biomolecules will be collected. That is, the working electrode defines the collecting area, which is at a surface of the working electrode in contact with the volume of the chamber.

Alternatively, the flow cell of the present invention may be an HGMS-based device.

In the HGMS-based device, a matrix having a suitable magnetic susceptibility is provided in the chamber such that when a magnetic field is applied across the chamber, a high magnetic field gradient is induced locally in the chamber, particularly in a space close to the surface of the matrix. This approach, referred to as *high gradient magnetic separation* (HGMS), allows capturing even weakly magnetized particles.

Preferably, a volume of the chamber of the HGMS-based device ranges from 1,000 mm³ to 10 m³, more preferably 0.01 to 100 L, and even more preferably 0.1 to 10 L.

Preferably, the matrix is disposed substantially along a central axis of the chamber. The matrix preferably comprises a ferromagnetic material.

Preferably, the matrix of the HGMS-based device is also used as the working electrode. The matrix thus similarly defines the collecting area, which is at the surface of the working electrode in contact with the volume of the chamber. The HGMS-based device preferably further comprises a counter electrode. The counter electrode may be disposed on the chamber. That is, a part or an entirety of the inner wall of the chamber may function as the counter electrode.

Other configurations of the HGMS-based device are possible, and the arrangement of the working electrode and the counter electrode is not limited to the above description. For example, the HGMS-based device may be a rotor-stator HGMS, and in such a scenario the counter electrode may be set either on the chamber wall or on the static disks. The matrix may be a composite material such as sandwich-like matrices of ferromagnetic and non-ferromagnetic materials, and in such a scenario the working electrodes may be set on the ferromagnetic matrices and the non-ferromagnetic matrices can be the counter electrode.

The general aspects of the present invention being described above, specific, non-limiting embodiments for further illustrating the present invention will be described below with reference to the respective drawings, in which:
Fig. 1 is a schematic diagram of a flow cell in accordance with a first embodiment of the present invention; and
Fig. 2 is a schematic diagram of a flow cell in accordance with a second embodiment of the present invention.

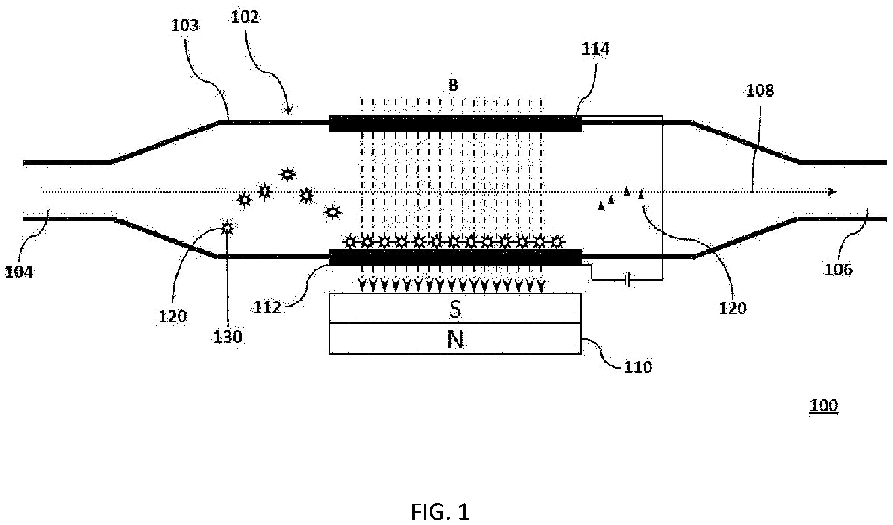

Referring to Fig. 1, a flow cell 100 in accordance with a first embodiment of the present invention is a microfluidic chip for separating biomolecules 120 from a liquid medium (not shown). The biomolecules 120 have bound to magnetic nanoparticles 130 before they are introduced together into the flow cell 100.

The flow cell 100 comprises a chamber 102. The chamber 102 comprises an inlet 104 and an outlet 106 disposed at respective ends along a length direction thereof. The inlet 104 and the outlet 106 define therebetween a fluid path 108. When a liquid medium is introduced into the chamber 102, it flows generally along the fluid path 108. The chamber 102 comprises a volume for containing the liquid medium, which is defined by a wall 103 of the chamber 102 and further by the inlet 104 and the outlet 106.

The flow cell 100 further comprises a working electrode 112 and a counter electrode 114. The working electrode 112 is disposed on the wall 103 of the chamber 102. The counter electrode 114 is disposed also on the wall 103 of the chamber 102, opposite to the working electrode 112. An electric potential difference may be established across the working electrode 112 and the counter electrode 114.

A magnetic source 110 is provided in proximity of the chamber 102, adjacent to the working electrode 112. The magnetic source 110 is a permanent magnet movably coupled to the chamber 102.

As shown in Fig. 1, the working electrode 112 is in contact with the volume containing the liquid medium. Therefore, the working electrode 112 defines a collecting area roughly at an interface between the working electrode 112 and the volume containing the liquid medium, i.e., the space occupied by the magnetic nanoparticles 130 in combination with the biomolecules 120.

The magnetic source 110, when placed in proximity of the chamber 102 as shown in Fig. 1, generates a magnetic field B extending at least between the collecting area and a rest of the volume of the chamber 102. Accordingly, when the liquid medium is contained in the chamber 102, the magnetic nanoparticles 130 bound with the biomolecule 120 are attracted by the magnetic field B toward the collecting area (or toward the working electrode 112) and immobilized in the collecting area.

The working electrode 112 is capable of generating an electric field extending at the collecting area. Thereby, after the magnetic nanoparticles 130 bound with the biomolecule 120 are collected on the working electrode 112 (or within the collecting area), an electric potential is applied at the surface of the working electrode 112 to break up the binding between the biomolecules 120 and the magnetic nanoparticles 130, releasing the biomolecules 120 whilst maintaining the magnetic nanoparticles 130 on the working electrode 112. The biomolecules 120 flowing out from the chamber 102 are collected, for example, in a collecting unit (not shown) in fluid communication with the outlet 106.

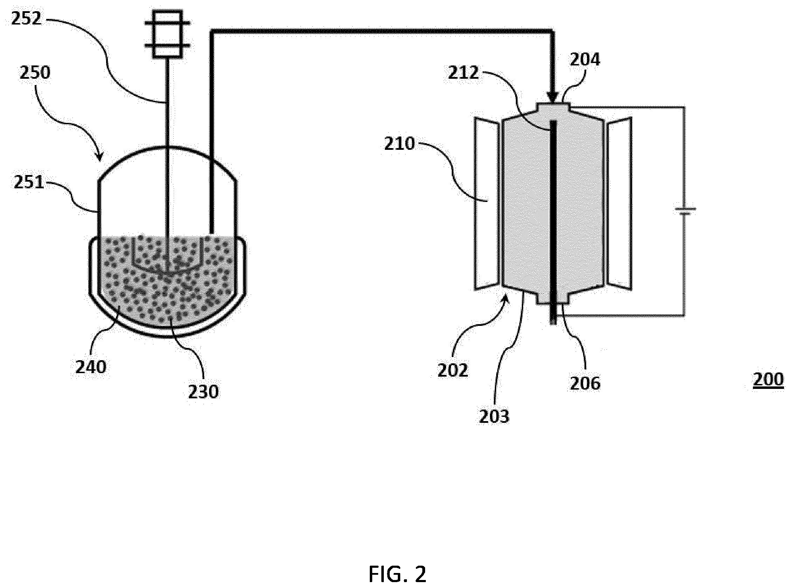

Referring to Fig. 2, a flow cell 200 in accordance with a second embodiment of the present invention is an HGMS-based device. The HGMS-based device comprises a chamber 202 and a matrix 212 disposed in the chamber 202, substantially along a central axis thereof. The chamber 202 comprises a volume, defined by the wall 203 of the chamber, for containing a liquid medium from which biomolecules are to be separated. The HGMS-based device further comprises a magnetic source 210 disposed in a proximity of the chamber 202, configured to apply a magnetic field to the liquid medium contained in the chamber 202.

The chamber 202 comprises an inlet 204 and an outlet 206. The inlet 204 is in fluid communication with a supply unit 250 for supplying the liquid medium. The supply unit 250 comprises a container 251 for containing the liquid medium and a stirrer 252. Here, the liquid medium comprises cell lysate 240 into which magnetic nanoparticles 230 are added. The cell lysate 240 comprises proteins that are fused with peptide tags having strong affinity with the magnetic nanoparticles 230. The stirrer 252 is used to stir the liquid medium to achieve a uniform dispersion. The stirring further helps the combination of the proteins with the magnetic nanoparticles 230.

The cell lysate 240 together with the magnetic nanoparticles 230, i.e. the liquid medium, are introduced into the chamber 202 via the inlet 204. In the meantime, the outlet 206 is closed so that the chamber 202 contains the liquid medium in its volume. The magnetic source 210 then applies a magnetic field across the chamber 202. High-gradient magnetic fields are created in the vicinity of the matrix 212. These fields extend at least from the volume of the chamber to the matrix 212. Therefore, the magnetic nanoparticles 230 will be attracted to the matrix 212. That is, a surface of the matrix 212 defines a collecting area for the magnetic nanoparticles 230 (or for the biomolecules).

The matrix 212 also functions as a working electrode. As shown in Fig. 2, a counter electrode is set on the chamber wall 203. Thereby, the working electrode and the counter electrode are capable of generating an electric field directing from the collecting area to the rest of the volume of the chamber (or vice versa). After the magnetic nanoparticles 230 are collected on the matrix 212, an electric potential is applied to the matrix 212 to repulse the biomolecules from the magnetic nanoparticles 230. The outlet 206 is then opened to allow the biomolecules to flow out and then be collected.

A skilled person will appreciate that many changes may be made to the details of the above-described embodiments and implementations without departing from the underlying principles thereof. The scope of the present invention should, therefore, be determined by the following claims.

## Claims

1. A method for separating biomolecules (120) from a liquid medium, comprising:
adding magnetic nanoparticles (130) to the liquid medium comprising the biomolecules, the biomolecules each adapted to bind to respective surfaces of the magnetic nanoparticles, the biomolecules comprising proteins fused with peptide tags;
bringing the liquid medium to which the magnetic nanoparticles have been added into contact with a collector (112);
applying a magnetic field (B) to the liquid medium in contact with the collector to attract the magnetic nanoparticles bound with the biomolecules to a surface of the collector; and
applying an electric potential to the surface of the collector to release the biomolecules from the magnetic nanoparticles,
wherein bringing the liquid medium to which the magnetic nanoparticles have been added into contact with the collector comprises:
allowing the liquid medium to which the magnetic nanoparticles have been added to pass through a flow cell (100, 200) comprising a chamber (102, 202) and the collector, the collector being a working electrode disposed in the chamber.

2. The method of claim 1, wherein the method further comprising attaching the peptide tags to the proteins.

3. The method of claim 2, wherein the peptide tags comprise charged peptide tags, preferably negatively charged peptide tags.

4. The method of claim 3, wherein an electrostatic repulsion is created between the magnetic nanoparticles and the charged peptide tags upon the application of the electric potential.

5. The method of any of claims 2-4, wherein the peptide tags form charge transfer complexes with the respective surfaces of the magnetic nanoparticles, the complex formation being breakable by a change of electrostatic interactions.

6. The method of any of claims 1-5, further comprising collecting the biomolecules released from the magnetic nanoparticles.

7. The method of any of the preceding claims, further comprising, before the application of the electric potential,
separating the collector from the liquid medium; and
bringing at least part of the collector into contact with another liquid medium.

8. The method of any of the preceding claims, wherein the magnetic nanoparticles comprise iron oxide nanoparticles, preferably superparamagnetic iron oxide nanoparticles (SPION).

9. The method of any of claims 1-8, wherein the peptide tags comprise negatively charged peptide tags and the method comprises applying a negative electric potential to the collector to repulse the proteins fused with the negatively charged peptide tags, thereby releasing the biomolecules from the magnetic nanoparticles.

10. The method of any of the preceding claims, further comprising removing the magnetic field to release the magnetic nanoparticles from the collector, the method preferably further comprising collecting the magnetic nanoparticles released from the collector.

11. A flow cell (200) for separating biomolecules from a liquid medium, the flow cell comprising:
a chamber (202) comprising
an inlet (204) and an outlet (206) which define therebetween a fluid path (108) for the liquid medium, and
a volume for containing the liquid medium provided along said fluid path, the volume comprising a collecting area for biomolecules;
a magnetic source (120) proximal to the volume of the chamber, the magnetic source arranged and configured to generate a magnetic field (B) extending at least between the collecting area and the remaining volume for containing the liquid medium; and
a working electrode (212) proximal to the volume for containing the liquid medium, the working electrode arranged and configured to generate an electric field at the collecting area,
wherein the flow cell is a high-gradient magnetic separation (HGMS)- based device (200), and a volume of the chamber ranges from 1,000 mm³ to 10 m³.

12. The flow cell of claim 11, wherein the magnetic source is a permanent magnet movably coupled to the working electrode and/or to the chamber or an electromagnet.

13. The flow cell of claim 11 or 12, further comprising
a first collecting unit in fluid communication with the outlet of the chamber for collecting the biomolecules from the liquid medium,
a second collecting unit in fluid communication with the outlet of the chamber for collecting a magnetic material from the liquid medium, and/or
a supply unit (250) in fluid communication with the inlet of the chamber for supplying the liquid medium to the chamber.

14. The flow cell of any of claims 11-13, wherein the working electrode is a matrix (212) of the HGMS-based device, the matrix being disposed substantially along a central axis of the chamber and comprising a ferromagnetic material, and the HGMS-based device further comprises a counter electrode disposed on the chamber.

## Patentansprüche

1. Verfahren zur Trennung von Biomolekülen (120) aus einem flüssigen Medium, aufweisend:
Hinzufügen von magnetischen Nanopartikeln (130) zu dem die Biomoleküle enthaltenden flüssigen Medium, wobei die Biomoleküle jeweils angepasst sind, um an jeweilige Oberflächen der magnetischen Nanopartikel zu binden, wobei die Biomoleküle mit Peptid-Tags fusionierte Proteine aufweisen;
In-Kontakt-Bringen des flüssigen Mediums, dem die magnetischen Nanopartikel hinzugefügt worden sind, mit einem Kollektor (112);
Anlegen eines Magnetfelds (B) an das flüssige Medium in Kontakt mit dem Kollektor, um die an die Biomoleküle gebundenen magnetischen Nanopartikel zu einer Oberfläche des Kollektors hin zu ziehen; und
Anlegen eines elektrischen Potentials an die Oberfläche des Kollektors, um die Biomoleküle von den magnetischen Nanopartikeln zu lösen,
wobei das In-Kontakt-Bringen des flüssigen Mediums, dem die magnetischen Nanopartikel hinzugefügt worden sind, mit dem Kollektor aufweist:
Hindurchfließenlassen des flüssigen Mediums, dem die magnetischen Nanopartikel hinzugefügt worden sind, durch eine Durchflusszelle (100, 200), die eine Kammer (102, 202) und den Kollektor aufweist, wobei der Kollektor eine in der Kammer angeordnete Arbeitselektrode ist.

2. Verfahren nach Anspruch 1, wobei das Verfahren ferner aufweist: Anbringen der Peptid-Tags an den Proteinen.

3. Verfahren nach Anspruch 2, wobei die Peptid-Tags geladene Peptid-Tags, vorzugsweise negativ geladene Peptid-Tags, aufweisen.

4. Verfahren nach Anspruch 3, wobei beim Anlegen des elektrischen Potentials eine elektrostatische Abstoßung zwischen den magnetischen Nanopartikeln und den geladenen Peptid-Tags hervorgerufen wird.

5. Verfahren nach einem der Ansprüche 2 bis 4, wobei die Peptid-Tags Ladungstransferkomplexe mit den jeweiligen Oberflächen der magnetischen Nanopartikel bilden, wobei die Komplexbildung durch eine Änderung elektrostatischer Wechselwirkungen brechbar ist.

6. Verfahren nach einem der Ansprüche 1 bis 5, ferner aufweisend Sammeln der von den magnetischen Nanopartikeln freigesetzten Biomoleküle.

7. Verfahren nach einem der vorstehenden Ansprüche, ferner vor dem Anlegen des elektrischen Potentials aufweisend:
Trennen des Kollektors von dem flüssigen Medium; und
In-Kontakt-Bringen mindestens eines Teils des Kollektors mit einem anderen flüssigen Medium.

8. Verfahren nach einem der vorstehenden Ansprüche, wobei die magnetischen Nanopartikel Eisenoxid-Nanopartikel, vorzugsweise superparamagnetische Eisenoxid-Nanopartikel (SPION) aufweisen.

9. Verfahren nach einem der Ansprüche 1 bis 8, wobei die Peptid-Tags negativ geladene Peptid-Tags aufweisen und das Verfahren aufweist: Anlegen eines negativen elektrischen Potentials an den Kollektor, um die mit den negativ geladenen Peptid-Tags fusionierten Proteine abzustoßen, um dadurch die Biomoleküle von den magnetischen Nanopartikeln freizusetzen.

10. Verfahren nach einem der vorstehenden Ansprüche, ferner aufweisend: Entfernen des Magnetfelds, um die magnetischen Nanopartikel von dem Kollektor freizusetzen, wobei das Verfahren ferner vorzugsweise aufweist: Sammeln der von dem Kollektor freigesetzten magnetischen Nanopartikel.

11. Durchflusszelle (200) zum Trennen von Biomolekülen aus einem flüssigen Medium, wobei die Durchflusszelle aufweist:
eine Kammer (202) mit:
einem Einlass (204) und einem Auslass (206), die zwischen sich einen Fluidpfad (108) für das flüssige Medium definieren, und
einem Volumen zum Aufnehmen des flüssigen Mediums, das entlang des Fluidpfads bereitgestellt ist, wobei das Volumen einen Sammelbereich für die Biomoleküle aufweist;
eine magnetische Quelle (120) proximal zu dem Volumen der Kammer, wobei die magnetische Quelle angeordnet und konfiguriert ist, um ein Magnetfeld (B) zu erzeugen, das sich zumindest zwischen dem Sammelbereich und dem das flüssige Medium enthaltenden restlichen Volumen erstreckt; und
eine Arbeitselektrode (212) proximal zu dem Volumen zum Aufnehmen des flüssigen Mediums, wobei die Arbeitselektrode angeordnet und konfiguriert ist, um ein elektrisches Feld an dem Sammelbereich zu erzeugen,
wobei die Durchflusszelle eine auf magnetische Trennung mit hohem Gradienten (HGMS) basierte Vorrichtung (200) ist und ein Volumen der Kammer von 1.000 mm³ bis 10 m³ reicht.

12. Durchflusszelle nach Anspruch 11, wobei die magnetische Quelle ein mit der Arbeitselektrode und/oder der Kammer bewegbar gekoppelter Permanentmagnet oder ein Elektromagnet ist.

13. Durchflusszelle nach Anspruch 11 oder 12, ferner aufweisend:
eine in Fluidverbindung mit dem Auslass der Kammer stehende erste Sammeleinheit zum Sammeln der Biomoleküle aus dem flüssigen Medium,
eine in Fluidverbindung mit dem Auslass der Kammer stehende zweite Sammeleinheit zum Sammeln eines magnetischen Materials aus dem flüssigen Medium, und/oder
eine in Fluidverbindung mit dem Einlass der Kammer stehende Zuführungseinheit (250) zum Zuführen des flüssigen Mediums zu der Kammer.

14. Durchflusszelle nach einem der Ansprüche 11 bis 13, wobei die Arbeitselektrode eine Matrix (212) der HGMS-basierten Vorrichtung ist, wobei die Matrix im Wesentlichen entlang einer zentralen Achse der Kammer angeordnet ist und ein ferromagnetisches Material aufweist, und die HGMS-basierte Vorrichtung ferner eine an der Kammer angeordnete Gegenelektrode aufweist.

## Revendications

1. Procédé de séparation de biomolécules (120) à partir d'un milieu liquide, comprenant :
l'addition de nanoparticules magnétiques (130) au milieu liquide comprenant les biomolécules, les biomolécules étant chacune adaptées pour se lier aux surfaces respectives des nanoparticules magnétiques, les biomolécules comprenant des protéines fusionnées avec des marqueurs peptidiques ;
la mise en contact du milieu liquide auquel les nanoparticules magnétiques ont été ajoutées avec un collecteur (112) ;
l'application d'un champ magnétique (B) au milieu liquide en contact avec le collecteur pour attirer les nanoparticules magnétiques liées avec les biomolécules vers une surface du collecteur ; et
l'application d'un potentiel électrique à la surface du collecteur pour libérer les biomolécules des nanoparticules magnétiques,
dans lequel la mise en contact du milieu liquide auquel les nanoparticules magnétiques ont été ajoutées avec le collecteur consiste à :
laisser le milieu liquide auquel les nanoparticules magnétiques ont été ajoutées passer à travers une cellule d'écoulement (100, 200) comprenant une chambre (102, 202) et le collecteur, le collecteur étant une électrode de travail disposée dans la chambre.

2. Procédé selon la revendication 1, dans lequel le procédé comprend de plus la fixation des marqueurs peptidiques aux protéines.

3. Procédé selon la revendication 2, dans lequel les marqueurs peptidiques comprennent des marqueurs peptidiques chargés, de préférence des marqueurs peptidiques négativement chargés.

4. Procédé selon la revendication 3, dans lequel une répulsion électrostatique est créée entre les nanoparticules magnétiques et les marqueurs peptidiques chargés lors de l'application du potentiel électrique.

5. Procédé selon l'une quelconque des revendications 2-4, dans lequel les marqueurs peptidiques forment des complexes de transfert de charge avec les surfaces respectives des nanoparticules magnétiques, la formation des complexes pouvant être rompue par une modification d'interactions électrostatiques.

6. Procédé selon l'une quelconque des revendications 1-5, comprenant de plus le recueil des biomolécules libérées des nanoparticules magnétiques.

7. Procédé selon l'une quelconque des revendications précédentes, comprenant de plus, avant l'application du potentiel électrique,
la séparation du collecteur du milieu liquide ; et
la mise en contact d'au moins une partie du collecteur avec un autre milieu liquide.

8. Procédé selon l'une quelconque des revendications précédentes, dans lequel les nanoparticules magnétiques comprennent des nanoparticules d'oxyde de fer, de préférence des nanoparticules d'oxyde de fer superparamagnétiques (SPION).

9. Procédé selon l'une quelconque des revendications 1-8, dans lequel les marqueurs peptidiques comprennent des marqueurs peptidiques négativement chargés et le procédé comprend l'application d'un potentiel électrique négatif au collecteur pour repousser les protéines condensées avec les marqueurs peptidiques négativement chargés, libérant par-là les biomolécules des nanoparticules magnétiques.

10. Procédé selon l'une quelconque des revendications précédente, comprenant de plus l'élimination du champ magnétique pour libérer les nanoparticules magnétiques du collecteur, le procédé comprenant de plus de préférence le recueil des nanoparticules magnétiques libérées du collecteur.

11. Cellule d'écoulement (200) pour séparer des biomolécules d'un milieu liquide, la cellule d'écoulement comprenant :
une chambre (202) comprenant
une entrée (204) et une sortie (206) qui définissent entre celles-ci une trajectoire de fluide (108) pour le milieu liquide, et
un volume pour contenir le milieu liquide fourni le long de ladite trajectoire de fluide, le volume comprenant une surface de recueil pour biomolécules ;
une source magnétique (120) proximale au volume de la chambre, la source magnétique disposée et configurée pour générer un champ magnétique (B) s'étendant au moins entre la surface de recueil et le volume restant pour contenir le milieu liquide ; et
une électrode de travail (212) proximale au volume pour contenir le milieu liquide, l'électrode de travail disposée et configurée pour générer un champ électrique sur la surface de recueil,
dans laquelle la cellule d'écoulement est un dispositif basé sur une séparation magnétique à gradient élevé (HGMS) (200), et un volume de la chambre est de 1 000 mm³ à 10 m³.

12. Cellule d'écoulement selon la revendication 11, dans laquelle la source magnétique est un aimant permanent couplé de manière amovible à l'électrode de travail et/ou à la chambre ou un électroaimant.

13. Cellule d'écoulement selon la revendication 11 ou 12, comprenant de plus
une première unité de recueil en communication fluide avec la sortie de la chambre pour recueillir les biomolécules du milieu liquide,
une seconde unité de recueil en communication fluide avec la sortie de la chambre pour recueillir un matériau magnétique du milieu liquide, et/ou
une unité d'alimentation (250) en communication fluide avec l'entrée de la chambre pour alimenter la chambre en le milieu liquide.

14. Cellule d'écoulement selon l'une quelconque des revendications 11-13, dans laquelle l'électrode de travail est une matrice (212) du dispositif basé sur HGMS, la matrice étant disposée substantiellement le long d'un axe central de la chambre et comprenant un matériau ferromagnétique, et le dispositif basé sur HGMS comprend de plus une contre électrode disposée sur la chambre.
